## Europäisches Patentamt

## European Patent Office

## Office européen des brevets

(19)

(11) Veröffentlichungsnummer: **0 206 360**
**B1**

(12)

# EUROPÄISCHE PATENTSCHRIFT

(45) Veröffentlichungstag der Patentschrift:
27.04.88

(21) Anmeldenummer: **86109574.3**

(22) Anmeldetag: **02.02.84**

(51) Int. Cl.⁴: **C 07 C 11/02,** C 07 C 1/20,
**B 01 J 29/04**

(54) **Verfahren zur Herstellung von C2- bis C4-Olefinen aus Methanol/Dimethylether.**

(30) Priorität: **10.02.83 DE 3304479**

(43) Veröffentlichungstag der Anmeldung:
**30.12.86 Patentblatt 86/52**

(45) Bekanntmachung des Hinweises auf die Patenterteilung:
**27.04.88 Patentblatt 88/17**

(84) Benannte Vertragsstaaten:
**BE DE FR GB NL**

(56) Entgegenhaltungen:
**EP-A-0 011 900**
**EP-A-0 022 640**

(73) Patentinhaber: **BASF Aktiengesellschaft, Carl-Bosch- Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Mross, Wolf Dieter, Dr., Anselm-Feuerbach- Strasse 21, D-6710 Frankenthal (DE)**
Erfinder: **Schwarzmann, Matthias, Dr., Carl- Bosch-Strasse 54, D-6703 Limburgerhof (DE)**

LIBER, STOCKHOLM 1988

**Beschreibung**

In neuerer Zeit gewinnen Bemühungen, Methanol zur Herstellung von Olefinen zu verwenden, zunehmendes Interesse. Methanol läßt sich aus Kohle, über Kohlevergasung und Merstellung von Synthesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff beibehalten werden. In den vergangenen Jahren sind daher Verfahren entwickelt worden, die die Herstellung von Olefinen aus Methanol und/oder Dimethylether zum Gegenstand haben.

Ein solches Verfahren ist beispielsweise in der OE-OS-2 615 150 beschrieben. Als Katalysator wird ein Aluminosilikatzeolith ZSM-5 verwendet, der eigentlich ein Aromatisierungskatalysator ist. Die Umsetzung kann aber durch verschiedene Maßnahmen, insbesondere durch Verkürzung der Verweilzeit, in Richtung Olefinbildung gelenkt werden. Weitere die Olefinbildung begünstigenden Parameter sind insbesondere die Verdünnung von Methanol bzw. Dimethylether mit Inertgasen bzw. Wasserdampf. Die Erfahrung zeigt, daß hohe Olefinausbeuten nur durch eine sehr starke Verdünnung mit Inertgas oder Wasserdampf zu erzielen sind. Andere bekannte Verfahren haben als Nachteil eine geringe Belastbarkeit und eine schnelle Verkokung des Katalysators. Die Verdünnung des Katalysators mit Bindemittel soll ebenfalls eine für die Olefinbildung vorteilhafte Maßnahme sein, doch werden durch die verwendeten Binder Nebenreaktionen und auch Desaktivierung des Katalysators verursacht.

Aus EP-A-22 640 war ein Verfahren zur Herstellung von Ethylen und höheren Kohlenwasserstoffen durch Abspaltung von Wasser aus Ethanol bekannt. Dabei entsteht allein Ethylen und daneben größere Mengen von höheren Kohlenwasserstoffen, deren Bildung im vorliegenden Falle verhindert werden soll. In den älteren Anmeldungen EP-A-72 920 und EP-A-110 255 wird jeweils Boehmit als Bindemittel vor der Verformung zugegeben.

Es wurde nun gefunden, daß man $C_2$- bis $C_4$-Olefine in hoher Ausbeute aus Methanol und/oder Dimethylether durch katalytische Umsetzung bei erhöhter Temperatur in Gegenwart von Borosilikat-Katalysatoren erhält, wenn man als Katalysatoren Borosilikatzeolithe verwendet, die ohne Bindemittel tablettiert oder verstrangt und mit Salzsäure behandelt wurden.

Ein bevorzugtes Verfahren zur Herstellung der erfindungsgemäg verwendeten Katalysatoren besteht darin, die nach der Synthese in der Ammonium-Form vorliegenden Borosilikatzeolithe durch Calcination bei 540°C/16 h in die acide H-Form überzuführen, und dieses calcinierte Produkt einer anschließenden Salzsäurebehandlung mit 3 bis 25 %-iger, insbesondere mit 10 unterwerfen. Das hierbei erhaltene Produkt wird abfiltriert, ausgewaschen bis keine Cl-Ionen mehr im Waschwasser nachweisbar sind, getrocknet bei 100°C bis 140°C und bei 500°C bis 600°C/5 h calciniert.

Die Säurebehandlung ist wesentlich für die Reaktionsfähigkeit des Katalysators; der reine unbehandelte Borosilikatzeolith reagiert erst bei Temperaturen oberhalb 550°C bzw. wenn Olefine zu- oder zurückgeführt werden bzw. reiner Dimethylether eingesetzt wird. Die hierbei erzielten Olefinausbeuten sind jedoch schlechter als bei den Katalysatoren mit Bindemittel unter gleichen Bedingungen. Die Säurebehandlung ermöglicht die Chemiesorption des Methanols am reinen Borosilikatzeolith und die Dehydration zu Dimethylether, dessen Bildung eine Voraussetzung für die Reaktion ist.

Bei der Durchführung des Verfahrens wird Methanol, das mit Dimethylether im Gleichgewicht steht, bei einem Druck zwischen Normaldruck und etwa 30 bar, vorzugsweise bei 0 bis 1 bar und bei Temperaturen zwischen 300°C und 650°C, vorzugsweise bei 400°C bis 550°C, an den oben beschriebenen Katalysatoren umgesetzt. Das Methanol kann einen Wassergehalt bis zu 90 Gew.-% haben, zweckmäßig verwendet man als Ausgangsstoff Rohmethanol, das etwa 20 % Wasser enthält.

Dem Methanol können auch noch andere niedere Alkohole beigemischt sein. Die Belastung des Katalysators, ausgedrückt in WHSV = $h^{-1}$ - g Methanol und/oder Dimethylether pro g Katalysator und Stunde-, wird zweckmäßig so gewählt, daß die Ausgangsstoffe möglichst quantitativ umgesetzt werden, so daß keine Abtrenn- und Rückführprobleme für nicht umgesetzten Dimethylether entstehen. Im allgemeinen soll daher die WHSV im Bereich von 0,5 bis 50 $h^{-1}$, vorzugsweise im Bereich von 2 bis 15 $h^{-1}$ liegen.

Durch das erfindungsgemäße Verfahren wird die Olefinselektivität im $C_2$-bis $C_4$-Bereich bei der Methanolumwandlung zu Kohlenwasserstoffen, insbesondere im Temperaturbereich von 400 bis 600°C wesentlich erhöht.

Die unerwünschten Nebenprodukte Methan und Aromaten, deren Bildung teilweise vom Bindemittel verursacht werden, werden durch Verwendung des erfindungsgemäßen Katalysators stark zurückgedrängt. Diese Tatsache äußert sich vorteilhaft; es wird eine Erhöhung der Laufzeit des eingesetzten Katalysators bewirkt.

Unter Laufzeit versteht man die Zeit zwischen Regenerierungen. Auch die Lebensdauer des Katalysators wird insgesamt verlängert. Der erfindungsgemäße Effekt der Laufzeitverbesserung wirkt sich besonders bei der Umsetzung bei hohen Temperaturen, z. B. im Bereich von 450 bis 550°C, aus.

Auch ist es von wirtschaftlicher Bedeutung, daß eine Verstrangung oder Tablettierung des Borosilikatzeolithen mit Bindemittel unterbleibt. Es ist ein weiterer Vorteil der Erfindung, daß man die Umsetzung zu $C_2$- bis $C_4$-Olefinen mit Rohmethanol ohne weiteren Zusatz von inerten Verdünnungsmittel wie $N_2$, He oder $H_2O$ ausführen kann.

Die Durchführung des erfindungsgemäßen Verfahrens wird anhand der nachfolgenden Beispiele näher erläutert.

## Beispiel

Der Bor-Zeolith wird in einer hydrothermalen Synthese aus hochdispersen 64 g $SiO_2$, 12,2 g $H_3BO_3$, 800 g einer wäßrigen 1,6-Hexandiamin-Lösung (Mischung 50 : 50) bei 170°C unter autogenem Druck in einem Rührautoklaven hergestellt. Nach Abfiltrieren und Auswaschen wird das kristalline Reaktionsprodukt bei 110°C/24 h getrocknet und bei 500°C/24 h calciniert. Dieser Borosilikatzeolith setzt sich zusammen aus 94,2 Gew.-% $SiO_2$ und 2,32 Gew.-% $B_2O_3$.

## Katalysator A (erfindungsgemäß)

50 g dieses Borosilikatzeolithen - in tablettierter oder verstrangter Form - werden mit 250 ml 18 %-iger Salzsäure 1 h bei 80°C behandelt, danach abfiltriert und mit Wasser chloridfrei gewaschen. Dieses Produkt wird bei 110°C/16 h getrocknet und bei 500°C/5 h calciniert.

Zum Ausbeutevergleich wurde der folgende Katalysator B herangezogen, der unter denselben Reaktionsbedingungen wie Katalysator A getestet wurde.

## Katalysator B

wird erhalten durch Verstrangen des oben beschriebenen Borosilikatzeolithen mit Boehmit im Verhältnis 60 : 40. Die Trocknung erfolgt bei 110°C/16 h und die Calcinierung bei 500°C/16 h.

| Katalysator | A | B |
|---|---|---|
| $C_2H_4$ | 6,7 | 12,0 |
| $C_3H_6$ | 42,9 | 32,9 |
| $C_4H_8$ | 20,9 | 16,1 |
| $CH_4$ | 1,2 | 7,9 |
| $C_2H_6$ | - | 0,6 |
| $C_3H_8$ | 0,6 | 2,1 |
| $C_4H_{10}$ | 1,0 | 1,2 |
| $C_5^+$-Aliphate | 20,3 | 9,4 |
| $C_6^+$-Aromaten | 5,6 | 15,9 |
| Laufzeit [h] | 12 | 7 |
| g $CH_3OH$/g Katalysator | 94 | 55 |

## Patentansprüche

1. Verfahren zur Herstellung von $C_2$- bis $C_4$-Olefinen durch katalytische Umsetzung von Methanol und/oder Dimethylether in Gegenwart von Borosilikatzeolithen bei erhöhter Temperatur, dadurch gekennzeichnet, daß man Borosilikatzeolithe verwendet, die ohne Bindemittel tablettiert oder verstrangt und mit Salzsäure behandelt wurden.

2. Verfahren nach Anspruch 1, dadurch gekennzeichnet, daß man die Salzsäurebehandlung mit 3 bis 25 %-iger Säure durchführt.

## Claims

1. A process for the preparation of a $C_2$-$C_4$-olefin by catalytic reaction of methanol andtor dimethyl ether in the presence of a borosilicate zeolite at elevated temperatures, wherein the borosilicate zeolite used has been pelletized or extruded without a binder and treated with hydrochloric acid.

2. A process as claimed in claim 1, wherein the hydrochloric acid treatment is carried out using from 3 to 25 % strength acid.

**Revendications**

1. Procédé de préparation d'oléfines en $C_2$ à $C_4$ par transformation catalytique de méthanol et/ou de diméthyléther en presence de zéalithes de borasilicate à temperature élevée, caractérisé en ce qu' on utilise des zéolithes de barosilicate qui ant été pastillées ou transformées en granulés cylindriques sans liant et qui ont été traitées par l'acide chlorhydrique.

2. Procédé selon la revendication 1, caractérisé en ce qu'on effectue le traitement par l'acide chlorhydrique avec un acide à 3-25 %.